# EUROPÄISCHE PATENTSCHRIFT

(11) **EP 3 980 109 B1**
(45) Veröffentlichungstag und Bekanntmachung des Hinweises auf die Patenterteilung: **10.09.2025**
(21) Anmeldenummer: 20733213.1
(22) Anmeldetag: 05.06.2020
(51) Int. Cl.: A61M 25/00

(54) **ABLAGEVORRICHTUNG FÜR MEDIZINISCHE FÜHRUNGSDRÄHTE ODER KATHETER SOWIE VERWENDUNG DAVON**
STORAGE DEVICE FOR MEDICAL GUIDE WIRES OR CATHETERS AND METHOD OF USE THEREOF
DISPOSITIF DE RANGEMENT POUR FILS-GUIDES OU CATHÉTERS MÉDICAUX ET PROCÉDÉ D'UTILISATION ASSOCIÉ

(30) Priorität: 07.06.2019 CH 7792019
(43) Veröffentlichungstag der Anmeldung: 13.04.2022
(73) Patentinhaber: Loumedic GmbH, 4565 Recherswil (CH)
(72) Erfinder: KARA, Levent, 8032 Zürich (CH); DELLA MORTE, Mario, 7130 Schnaus (CH)
(74) Vertreter: Frei Patent Attorneys
(86) Internationale Anmeldenummer: PCT/EP2020/065608
(87) Internationale Veröffentlichungsnummer: WO 2020/245344

(56) Entgegenhaltungen:
- WO-A1-2008/139852
- JP-A- 2012 070 905
- US-A- 4 332 322
- US-A1- 2014 144 798
- US-A1- 2017 333 668

## Beschreibung

Die Erfindung betrifft eine Ablagevorrichtung für medizinische Führungsdrähte oder Katheter sowie ihre Verwendung. Medizinische Führungsdrähte kommen in der Urologie, der interventionellen Radiologie, der Kardiologie, der Herzchirurgie, der Gefässchirurgie, der Gastroentrologie und in weiteren medizinischen Gebieten zur Anwendung. Sie dienen insbesondere der Führung und Stabilisierung von Kathetern bei deren Einführung. Führungsdrähte weisen unterschiedliche Steifigkeiten auf und können ausserdem recht lang sein, beispielsweise bis zu 3 m. Ihre Ablage und das sichere Verwahren vor und während eines entsprechenden medizinischen Eingriffs ist wegen der geforderten Flexibilität eine Herausforderung. Dasselbe gilt für die Ablage und das Verwahren von Kathetern, die ebenfalls in unterschiedlichen Längen und mit unterschiedlichen Steifigkeiten vorhanden sind.

Traditionell werden Führungsdrähte in einen mit etwas Salzlösung gefüllten Behälter abgelegt. Das ist angesichts der Eigensteifigkeit der Führungsdrähte nicht ganz unproblematisch und insbesondere auch dann unbefriedigend, wenn mehr als ein Führungsdraht benötigt wird und im Behälter liegt.

Beispielsweise aus der US 2012/0312703, der US 5,769,222 oder der WO 2008/139852 sind Behälter bekannt, welche mit Halteelementen versehen sind, durch welche in den Behältern abgelegte Führungsdrähte an dafür vorgesehenen Orten auf definierte Weise gehalten werden. Die Behälter sind zum Beinhalten einer Flüssigkeit ausgebildet. Auf dem Boden der Behälter werden die Führungsdrähte gewickelt abgelegt. Die Halteelemente bilden oberhalb der Führungsdrähte eine flanschartige Ausbördelung, deren Unterseite parallel zum Boden verläuft und die verhindert, dass die Führungsdrähte unbeabsichtigt aus der Halterung springen können.

Die Ablage sowie die Entnahme der gewickelten Führungsdrähte aus solchen Behältern können nur durch ein beidhändiges Eingreifen und nur mit einem gewissen Aufwand erfolgen. Bei der Ablage ist ausserdem die Grösse der Wicklungen durch die Konstruktion der Behälter recht eng vorgegeben, was gerade bei wiederholtem Gebrauch eines Führungsdrahts im Rahnen eines - unter Zeitdruck stattfindenden - Eingriffs nicht unproblematisch ist.

US 2014/144798 zeigt die Ablage eines eines Schlauchs, in welchem ein Führungsdraht mechanisch geschützt angeordnet ist. Die Ablage des Schlauchs erfolgt ungebündelt, entlang einer spiralförmigen Bahn.

US 4,332,322 zeigt einen Faltbehälter für sterile Schläuche. Der Faltbehälter weist herausfaltbare Strukturen auf, welche eine Aufnahme für die Schläuche bilden und so diese entsprechend fixieren können.

Die US 2017/0333668 zeigt eine Katheterpackung mit Begradigungsvorrichtung, durch welche der Katheter bei der Entnahme gezogen wird.

JP 2012-70905 zeigt Ablagevorrichtungen für chirurgische Drähte, unter anderem mit Bahnen, die 8-förmig sind.

Es ist Aufgabe der Erfindung, eine Ablagevorrichtung für medizinische Führungsdrähte oder Katheter vorzusehen, welche Nachteile des Standes der Technik überwindet und welche insbesondere eine einfache Handhabe bietet und für ein wiederholtes Ablegen und wieder Entnehmen eines oder mehrerer der Führungsdrähte oder Katheter besonders gut geeignet ist.

Gemäss einem Aspekt der Erfindung wird eine Ablagevorrichtung für medizinische Führungsdrähte oder Katheter zur Verfügung gestellt, gemäß Anspruch 1.

Generell gilt, dass eine Bahn im Sinne des vorliegenden Texts eine Bahn ist, welche von einer Drahtwicklung eingenommen wird, die aufgrund ihrer Eigensteifigkeit eine minimale Krümmung anstrebt, d.h. eine Drahtwicklung eines in sich steifen und im entspannten Zustand geraden Drahtes.

Die Konstruktion mit den genannten drei Anlageflächen hat eine Reihe von Vorteilen. Medizinische Führungsdrähte und Katheter tendieren aufgrund ihrer Eigensteifigkeit dazu, einem Biegen durch eine elastische Gegenkraft entgegenzuwirken. Daher ist es an sich bekannt, dass Führungsdrähte durch aussenseitige Führungsmittel begrenzt in Wicklungen abzulegen, die aufgrund der aussenseitigen Begrenzung eine ungefähr elliptische Form einnehmen. Konstruktionsgemäss geben entsprechende Ablagevorrichtungen gemäss dem Stand der Technik die erforderliche Grösse der Wicklung relativ genau vor. Das kann insbesondere für die Wieder-Ablage nach erstmaligem Gebrauch bei den oft zeitkritischen Bedingungen während eines Eingriffs nachteilig sein. Ausserdem benötigen Ablagevorrichtungen aus dem Stand der Technik relativ viel Platz. Schliesslich ist auch die Entnahme eines abgelegten Führungsdrahtes oder Katheters oft etwas diffizil, weil darauf geachtet werden muss, dass sich der Führungsdraht bzw. Katheter nicht irgendwo verheddert oder gar zu Boden fällt und deshalb nicht mehr verwendet werden kann. Die erfindungsgemässe Konstruktion ist im Gegensatz dazu sehr einfach und flexibel. Die Elastizität des aufgewickelten Führungsdrahtes oder Katheter bringt diesen dazu, eine möglichst kreisförmige Konfiguration anzustreben, weshalb er ab einer gewissen Wicklungs-Minimallänge aufgrund seiner Elastizität nach aussen gegen die erste und dritte Anlagefläche und gleichzeitig nach innen gegen die erste Anlagefläche drückt, weshalb er stabil gehalten wird. Dies gilt, sofern die Wicklungs-Minimallänge erreicht wird, unabhängig von der Grösse der Wicklung, funktioniert also für völlig unterschiedliche Wicklungslängen, wobei der gewickelte Führungsdraht oder Katheter je nach Länge der Wicklung und Eigensteifigkeit einer ungefähr elliptischen oder auch birnenförmigen Bahn folgt.

Auch die Entnahme ist besonders einfach. Ein Zusammendrücken der Führungsdraht-(oder Katheter-) Wicklung weg von der ersten und der dritten Anlagefläche löst den Führungsdraht bzw. Katheter gleich auch von der zweiten Anlagefläche (die Wicklung wird schmaler und dadurch automatisch länger), so dass der Führungsdraht oder Katheter mit einem einzigen Handgriff, unter Umständen gar einhändig entnommen werden kann.

Die Krümmung der Bahn kann ungefähr monoton sein. Die Bahn kann insbesondere ungefähr elliptisch sein. Eine ungefähr elliptische Bahn ergibt sich abhängig von der Länge der Wicklung aufgrund der Eigensteifigkeit des Führungsdrahtes oder Katheters von selbst, wenn die Führungsdraht-Wicklung (bzw. Katheter-Wicklung) seitlich an zwei Punkten (entsprechend der ersten und dritten Anlagefläche, wenn diese ungefähr punktförmig sind) zusammengedrückt wird; die innenliegende zweite Anlagefläche kann eine gewisse Abweichung vom elliptischen Verlauf bewirken. Die monotone Krümmung der Bahn schliesst nicht aus, dass der aufgewickelte Führungsdraht oder Katheter lokal am Ort des Anstehens an der jeweiligen aussenstehenden Anlagefläche aufgrund seiner Elastizität leicht nach innen ausgelenkt und also etwas abgeflacht oder gar lokal entgegen der Bahnkrümmung gekrümmt ist. Bei längeren Wicklungen kann die Bahn auch einen ungefähr birnenförmigen Verlauf einnehmen; die genaue sich ergebende Form ist die, bei welcher die in den Führungsdraht oder Katheter durch dessen Biegung auf die Bahn einzubringende potentielle Energie minimiert wird.

Bei einer Ablage des aufgewickelten Führungsdrahts oder Katheters entlang einer gekrümmten Bahn wird diese Eigensteifigkeit des Führungsdrahtes bzw. Katheters ausgenutzt, indem der aufgewickelte Führungsdraht bzw. Katheter dazu tendiert, eine Konfiguration mit möglichst geringer Krümmung, d.h. bei fester Wicklungslänge eine kreisförmige Konfiguration einzunehmen. Dies wird in der erfindungsgemässen Ablagevorrichtung genutzt, indem sich eine wie vorstehend erklärt eigenstabile Konfiguration ergibt, in welcher der Führungsdraht bzw. Katheter nach aussen gegen die erste und dritte Anlagefläche und nach innen gegen die zweite Anlagefläche gedrückt wird, unabhängig von der genauen Länge der Führungsdraht- bzw. Katheter-Wicklung.

Am Ort der Anlageflächen sowie ganz generell ist der Führungsdraht oder Katheter durch die Ablagevorrichtung jeweils nur einseitig geführt, im Gegensatz zu einer Führung in einem Führungskanal. Das heisst, gegenüberliegend der jeweiligen Anlageflächen ist kein die Bewegung des Führungsdrahts bzw. Katheters entlang der Ablageebene behinderndes Element vorhanden.

Die erste, zweite und dritte Anlagefläche kann insbesondere durch je ein von der Basisplatte abstehendes erstes, zweites und drittes Führungselement gebildet sein. Deren Anordnung ist dann so, dass die Bahn innenseitig am ersten Führungselement, aussenseitig am zweiten Führungselement und innenseitig am dritten Führungselement vorbeiführt.

Die Anordnung der Führungselemente definiert eine Vorderseite und eine Hinterseite der Ablagevorrichtung: das erste und das dritte Führungselement und die von ihnen gebildeten Anlageflächen sind vorderseitig angeordnet, das zweite Führungselement entsprechend hinterseitig.

Damit die Vorteile der Erfindung voll zur Geltung kommen, können insbesondere folgende optionale, miteinander kombinierbare Merkmale von Vorteil sein:
- Mindestens das zweite Führungselement (und gegebenenfalls auch ein weiteres, bspw. viertes Führungselement, s.u.), an welchem die Bahn aussenseitig vorbeiführt) ist in einem Abstand vom Rand der Basisplatte angeordnet, d.h. die Basisplatte erstreckt sich von diesem Führungselement aus und bspw. auch von den anderen Führungselementen aus in alle Richtungen.
- Die Basisplatte ist mindestens hinterseitig und/oder vorderseitig je frei von einer Wand, d.h. sie läuft am Rand aus. Durch das Fehlen einer vorderseitigen Begrenzung wird die Flexibilität der Vorrichtung in Bezug auf die Länge der Wicklung voll unterstützt. Das Fehlen einer hinterseitigen Begrenzung erleichtert das Hineinlegen und Entfernen des Drahtes bzw. Katheters, indem die von ihm gebildete Schlaufe nur entlang ihrer kleineren Achse entgegen ihrer Eigensteifigkeit zusammengedrückt werden muss, was die Ausdehnung entlang ihrer grösseren Achse leicht erhöht.
- Der Abstand der Führungselemente ist genügend gross um ein Ablegen ohne zu grosse Verformungen zu ermöglichen. Der Mindestabstand benachbarter Führungselemente beträgt bspw. mindestens 1.5 cm oder mindestens 2 cm oder mindestens 3 cm oder mindestens 4 cm. Ergänzend oder alternativ kann der Abstand grösser sein als ein durchschnittlicher horizontaler Durchmesser der entsprechenden Führungselemente, bspw. um mindestens einen Faktor 1.5 oder mindestens einen Faktor 2.
- Die Führungselemente sind als pilzartig von der Basisplatte abstehende und fest mit dieser verbundene Elemente ausgestaltet. Pilzartig heisst, dass sie sich ausgehend von einem dünneren Stamm nach oben, d.h. in Richtung von der Basisplatte weg, verdicken.
- Die Anlageflächen bilden für den Führungsdraht bzw. Katheter eine einseitige Anlage - d.h. die Führungselemente sind nicht für ein Klemmen oder dergleichen ausgebildet. Vielmehr ist die Vorrichtung so ausgebildet, dass nichts einem Wegbewegen des Führungsdrahtes bzw. Katheters in horizontaler Richtung von der Anlagefläche weg ist entgegensteht. In einer Umgebung jeder Anlagefläche (in horizontaler Richtung senkrecht von der Anlagefläche weg) ist die Basisplatte frei von nach oben vorstehenden Elementen, so dass das Anlegen sowie bestimmungsgemässe Entfernen der Wicklung auf die nachstehend noch eingehender beschriebene Art nicht behindert wird.

Insgesamt ist die Ablagevorrichtung insbesondere als in sich starrer Gegenstand ausgebildet, der die Basisplatte und die Mehrzahl (bspw. mindestens drei) davon senkrecht nach oben abstehenden, bspw. pilzartig ausgebildeten Führungselemente bildet, wobei die Basisplatte frei von einer Einsäumung ist. Dass die Ablagevorrichtung ein in sich starrer Gegenstand ist heisst auch, dass sie frei von beweglichen Elementen, bspw. Rollen oder dergleichen ist.

Die Ablagevorrichtung kann insbesondere aus der Basisplatte und den Führungselementen sowie einer eventuellen unterseitig auf der Basisplatte vorhanden Befestigungseinrichtung (Kleber oder dergleichen) bestehen, d.h. frei von weiteren Elementen sein. Die Ablagevorrichtung kann einstückig ausgebildet sein, bspw. als Kunststoffteil, bspw. als Spritzgussteil.

Die Anzahl der Führungselement beträgt bspw. mindestens drei oder mindestens fünf, beispielsweise genau fünf.Es wäre zwar an sich möglich, dass die erste und dritte Anlagefläche durch ein gemeinsames, bereichsweise um die Bahn herum verlaufendes Aussen-Führungselement gebildet wird. Die Ausgestaltung mit dedizierten, voneinander getrennten aussenseitigen erstem und drittem Führungselement ist aber besonders vorteilhaft. Erstens ist sie besonders materialsparend und trägt dem Umstand Rechnung, dass pro Anlagefläche ein im Wesentlichen punktkontaktartiges Anliegen der Führungsdraht-oder Katheter-Wicklung ausreicht, d.h. es braucht gar keine ausgedehnte Anlagefläche, sondern der gewickelte Führungsdraht oder Katheter berührt die Anlagefläche nur an jeweils einem Punkt. Zweitens sind das Ablegen und das Entfernen des medizinischen Führungsdrahts oder Katheters besonders einfach wenn Bewegungen des Führungsdrahts bzw. Katheters entlang der Ablageebene mit Ausnahme der Anlageflächen möglichst ungehindert möglich sind.

Aus einem ähnlichen Grund kann es vorteilhaft sein, wenn die Anlageflächen in einem Schnitt parallel zur Ablageebene konvex gekrümmt sind, was bei der ersten und dritten Anlagefläche bedeutet, dass sie entgegen der Krümmung der Bahn gekrümmt sind.

Besonders vorteilhaft sind die Führungselemente als pilzartig von der Basisplatte abstehende Elemente ausgebildet.

Durch den Überhang (d.h. Hinterschnitt in Bezug auf Richtungen senkrecht zur Ablageebene) wird bei jeder Anlagefläche eine beispielsweise hohlkehlenartige Vertiefung gebildet, in welche der aufgewickelte Führungsdraht oder Katheter aufgrund seiner Eigensteifigkeit gedrückt wird. In Ausführungsformen ist diese Vertiefung mindestens bei den vorderseitigen Anlageflächen (der ersten und der dritten Anlagefläche) in einem vertikalen Abstand von der Plattenoberseite angeordnet, d.h. der Führungsdraht oder Katheter ist oberhalb der Plattenebene geführt und kann so einfach untergriffen werden; der Abstand der Vertiefung zur Plattenoberseite kann bspw. bei den vorderseitigen Führungselementen mindestens ca. 0.6 cm oder mindestens ca. 1 cm betragen. Zu diesem Zweck weist das entsprechende Führungselement eine nach unten auslaufende Partie auf.

Im Schnitt senkrecht zur Ablageebene kann das Führungselement insbesondere konkav gekrümmt sein, so dass sich die genannte Hohlkehle bildet und der Führungsdraht oder Katheter eine definierte vertikale Position erhält.

Die Ablagevorrichtung kann insbesondere auch zur örtlich getrennten Ablage von mehr als einem Führungsdraht oder Katheter (das beinhaltet die Möglichkeit, je einen Führungsdraht und einen Katheter abzulegen) ausgebildet sein. Zu diesem Zweck kann die Vorrichtung eine vierte, fünfte und sechste Anlagefläche ausbilden.

Dabei können bspw. die dritte und vierte Anlagefläche durch ein gemeinsames Führungselement gebildet sein. Ein solches gemeinsames Führungselement kann senkrecht zur Bahnrichtung eine grössere Ausdehnung hat als Führungselemente mit nur einer Anlagefläche. Jedenfalls kann der Abstand der Anlageflächen so gewählt sein, dass die Bahnen benachbarter abgelegter Drähte oder Katheter nicht überlappen. In diesem Kontext kann auch vorteilhaft sein, dass die Hauptachsen der benachbarten Bahnen einen Winkel zueinander bilden, so dass sie nach hinten, also von den Anlageflächen weg, voneinander weg verlaufen.

Die definierte Position der Bahnen benachbart abgelegter Führungsdrähte oder Katheter, gegebenenfalls auch in vertikaler Richtung, hilft insbesondere zu verhindern, dass bei Entnahme eines der Führungsdrähte oder Katheter der benachbarte abgelegte Führungsdraht oder Katheter unbeabsichtigt aus der Befestigung gehoben wird.

In Ausführungsformen weist die Ablagevorrichtung auf der Plattenunterseite einen Kleber auf. Dadurch kann sie an einer Oberfläche (Arbeitsfläche) eines Arbeitstischs befestigt werden. Dieses an sich im Nachhinein simpel erscheinende Mittel bringt einen wichtigen konstruktiven Vorteil mit sich. Es ermöglicht, dass nicht die ganze Wicklung des Führungsdrahts oder Katheters auf der Basisplatte abgelegt werden muss. Vielmehr kann sich der Führungsdraht oder Katheters über die Arbeitsfläche des Arbeitstischs erstrecken. Daher kann die Ablagevorrichtung als Ganze ohne Beeinträchtigung ihrer Funktionalität relativ klein und kompakt ausgestaltet sein.

Die Ablagevorrichtung kann wie bereits vorstehend angedeutet insbesondere im Gegensatz zu Vorrichtungen aus dem Stand der Technik so ausgestaltet sein, dass sie nicht behälterartig mit umlaufenden seitlichen Wänden ist. Vielmehr ist die Ablagevorrichtung in Ausführungsformen insbesondere nach vorne, d.h. zur Seite weg vom die zweite Anlagefläche bildenden Führungselement, offen, so dass die Länge der Wicklung nicht beschränkt ist.

In Ausführungsformen bildet die Basisplatte als Ganze einen Bogen, d.h. sie ist in der Ablageebene gekrümmt, und zwar so, dass wie vorstehend erwähnt die Achsen der Bahnen einen Winkel zueinander bilden und nach hinten, also von den Anlageflächen weg, voneinander weg verlaufen.

Die Ablagevorrichtung kann mindestens im Bereich der Basisplatte und beispielsweise als Ganze transparent sein.

Die Erfindung betrifft nebst der hier beschriebenen Ablagevorrichtung in den erwähnten Gestaltungsmöglichkeiten auch ihre Verwendung zur Ablage eines medizinischen Führungsdrahtes oder Katheters wie im Anspruch 13 definiert. Dieser ist insbesondere für medizinische Eingriffe zugelassen und aus einem für medizinische Eingriffe zugelassenen Material gefertigt, bspw. aus Edelstahl (blank oder Beschichtet, bspw. mit PTFE) oder aus Nitinol (blank oder beschichtet, bspw. mit PTFE). Die Ablage erfolgt bestimmungsgemäss, durch Aufwickeln des Führungsdrahts oder Katheters, leichtes seitliches Zusammenpressen der Wicklung bis der Führungsdraht bzw. Katheter zwischen die erste und dritte Anlagefläche eingefügt werden kann, mit dem die zweite Anlagefläche bildenden Führungselement auf der Innenseite der Wicklung, und Loslassen in eine eigenstabile Konfiguration, in welcher die Wicklung aufgrund ihrer Eigensteifigkeit nach aussen an die erste und dritte Anlagefläche und nach innen an die zweite Anlagefläche gedrückt wird. In Ausführungsformen mit vierter, fünfter und sechster Anlagefläche gilt Entsprechendes auch für eine zweite Wicklung, welche durch leichtes seitliches Zusammenpressen der Wicklung Katheter zwischen die vierte und sechste Anlagefläche eingefügt werden kann, mit dem die fünfte Anlagefläche bildenden Führungselement auf der Innenseite der Wicklung und loslassen ein eine eigenstabile Konfiguration, in welcher die Wicklung aufgrund ihrer Eigensteifigkeit nach aussen an die vierte und sechste Anlagefläche und nach innen an die fünfte Anlagefläche gedrückt wird.

Um eine bestimmungsgemässe Verwendung zu gewährleisten kann die Ablagevorrichtung in einem Set zur Verfügung gestellt werden, welches nebst der Ablagevorrichtung auch Informationen enthält - bspw. in Papierform oder elektronisch - in der die erwähnte bestimmungsgemässe Ablage gelehrt wird und insbesondere die Anordnung der Wicklung in Bezug auf die erste, zweite und dritte Anlagefläche (und ggf. vierte, fünfte und sechste Anlagefläche)

Eine bestimmungsgemässe Verwendung beinhaltet auch die Möglichkeit, eine zweite oder bspw. auch eine dritte Ablagevorrichtung hinzuzufügen und also mehrere Ablagevorrichtungen auf ein- und derselben Arbeitsfläche (Arbeitstisch oder dergleichen) zu verwenden, beispielsweise nebeneinander.

Nachfolgend werden Ausführungsformen der Erfindung anhand von Zeichnungen genauer beschrieben. In den Figuren bezeichnen gleiche Bezugszeichen gleiche oder analoge Elemente. Es zeigen:
- Figur 1 eine Ansicht einer erfindungsgemässen Ablagevorrichtung;
- Fig. 2 eine Ansicht der Ablagevorrichtung von Fig. 1 mit eingezeichneten möglichen ersten und zweiten Bahnen;
- Fig. 3 eine Seitenansicht der Ablagevorrichtung nach Fig. 1; und
- Fig. 4 eine weitere Ansicht.

In der nachstehenden Beschreibung eines von Ausführungsbeispielen ist die Anwendung für die Ablage von medizinischen Führungsdrähten illustriert. Die gezeichnete und beschriebene Ablagevorrichtung kann aber auch für die Ablage eines Katheters (eventuell in Kombination mit der Ablage eines Führungsdrahtes) oder von mehreren Kathetern verwendet werden.

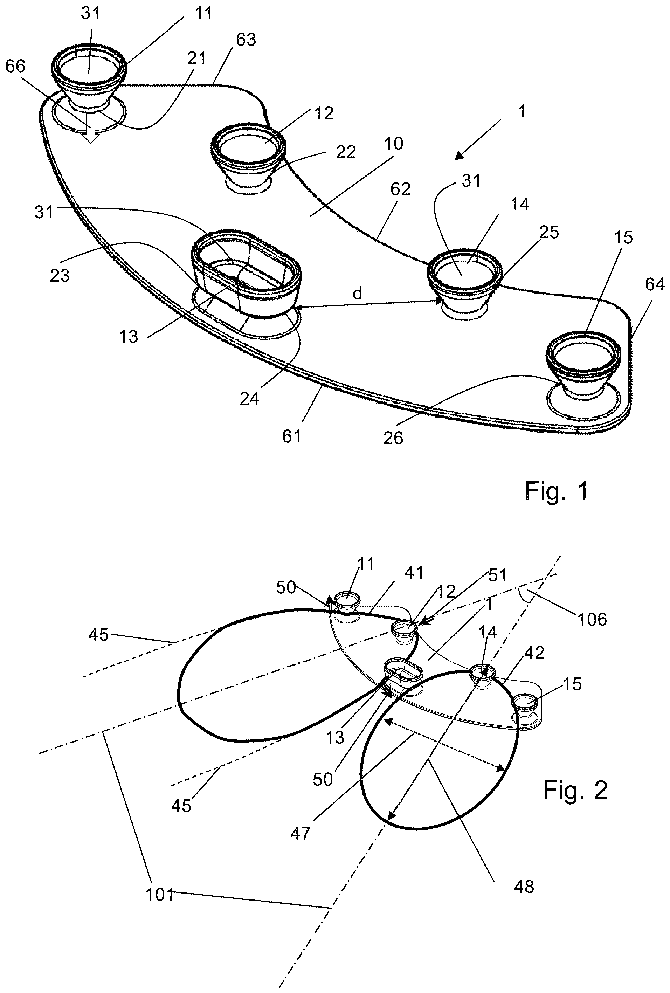

**Figur 1** zeigt eine Ansicht einer erfindungsgemässen Ablagevorrichtung 1. Diese weist eine Basisplatte 10 auf, welche oberseitig eine Ablageebene für einen oder mehrere Führungsdrähte definiert. Von der Basisplatte abstehend sind insgesamt fünf Vorsprünge ausgebildet, welche das erste, zweite, dritte, vierte und fünfte Führungselement 11, 12, 13, 14 bzw. 15 bilden. Die Führungselemente 11-15 sind jeweils so ausgebildet, dass sie mindestens im Bereich einer Anlagefläche einen Überhang ausbilden und damit das Abgleiten eines an die Anlagefläche anstehenden Drahtes nach oben verhindern.

Insgesamt ist die Ablagevorrichtung als Platte mit einer diskreten Anzahl von pilzartig von der Platte abstehenden Führungselementen gefertigt. Sie ist frei von einer umlaufenden Wand, d.h. frei von einer Einsäumung.

Die Anordnung der Führungselemente definiert eine Vorder- und eine Hinterseite, wobei das erste, dritte und fünfte Führungselement 11, 13, 15 vorderseitig und das zweite und vierte Führungselement in Bezug darauf hinterseitig angeordnet sind Die Führungselemente sind im Abstand von den Rändern 61, 62, 63, 64 angeordnet, wobei insbesondere ein Abstand des zweiten und vierten Führungselements 12, 14 vom hinterseitigen Rand 62 von Bedeutung ist. Ebenso von Bedeutung ist, dass mindestens vorder- hinterseitig keine Wände vorhanden sind, damit die Wicklungen gut abgelegt und wieder entfernt werden können.

Die Führungselement sind fest und starr mit der Basisplatte verbunden, damit die Ablagevorrichtung stets stabil ist und auch bei Arbeit unter Zeitdruck und möglichen unbedarften Bewegungen nichts verschieben kann. Insgesamt ist die Ablagevorrichtung insbesondere als monolithischer Gegenstand, bspw. aus Kunststoff gefertigt, beispielsweise als spritzgegossenes Teil.

Die Abstände d benachbarter Führungselemente - in Fig. 1 ist exemplarisch der Abstand d zwischen dem dritten und vierten Führungselement eingezeichnet - sind so gewählt, dass die nachstehend näher beschriebene Ablage möglich ist. Sie betragen typischerweise einige cm, insbesondere mindestens 2 cm.

**Figur 2** illustriert je eine von der Ablagevorrichtung definierte erste Bahn 41 und zweite Bahn 42, entlang welcher je ein aufgewickelter Führungsdraht abgelegt werden kann. Die Wicklungen eines solchen Führungsdrahts verlaufen ungefähr entlang der jeweiligen Bahn 41, 42, wobei in Fig. 2 auch zwei Führungsdraht-Endstücke 45 angedeutet sind. Der genaue Verlauf der Bahnen 41, 42 hängt von der Grösse der Wicklungen ab. Wie in Fig. 2 durch die unterschiedlichen Abmessungen der beiden Bahnen 41, 42 angedeutet können die Wicklungen unterschiedlich gross sein. Aufgrund des erfindungsgemässen Ansatzes werden Wicklungen in einem recht substantiellen Grössenbereich stabil gehalten. Die Elastizität des aufgewickelten Führungsdrahtes bringt diesen dazu, eine möglichst kreisförmige Konfiguration anzustreben, weshalb er ab einer gewissen Wicklungs-Minimallänge aufgrund seiner Elastizität nach aussen gegen das erste und dritte Führungselement 11, 13 (siehe Doppelpfeile 50) beziehungsweise das dritte und fünfte Führungselement 13, 15 sowie nach innen gegen das zweite (Doppelpfeil 51) beziehungsweise vierte Führungselement 12, 14 gedrückt wird und so auf stabil auf einen Verlauf entlang der Bahn gezwungen wird.

In Fig. 2 ist die hier etwas längere erste Bahn 41 leicht birnenförmig dargestellt, während die etwas kürzere zweite Bahn 42 näherungsweise elliptisch ist. In beiden Fällen kann - das ist generell für verschiedene Ausführungsformen der Erfindung der Fall - das zweite Führungselement 12 bzw. vierte Führungselement 14 ungefähr in der mittelsenkrechten Ebene zwischen den Berührungspunkten an die erste und die dritte Anlagefläche 21, 23 bzw. vierte und sechste Anlagefläche 24, 26 angeordnet sein und so die Bahn ungefähr symmetrisch zu dieser mittelsenkrechten Ebene verlaufen; diese Symmetrie definiert auch je eine Achse 101 der beiden Bahnen 41, 42.

Es bilden sich also, wieder bezugnehmend auf Fig. 1, Anlageflächen die so angeordnet sind, dass die erste Bahn 41 am ersten Führungselement innenseitig (erste Anlagefläche 21), am zweiten Führungselement aussenseitig (zweite Anlagefläche 22) und am dritten Führungselement innenseitig (dritte Anlagefläche 23) vorbei führt. Analog ist die Anordnung der Anlageflächen für die zweite Bahn 42 so, dass die zweite Bahn 42 am dritten Führungselement 13 innenseitig (vierte Anlagefläche 24), am vierten Führungselement 14 aussenseitig (fünfte Anlagefläche 25) und am fünften Führungselement 15 wieder innenseitig (sechste Anlagefläche 26) vorbei führt. Umgekehrt betrachtet befinden sich das erste, dritte und fünfte Führungselement jeweils aussenseitig der ungefähr parallel zur Ablageebene verlaufenden Bahnen (wobei die Innen- und Aussenseite durch die Krümmung der Bahnen definiert werden), so dass eine Aussenseite der jeweiligen Wicklung daran ansteht, und das zweite und das vierte Führungselement ist innenseitig angeordnet, so dass eine Innenseite der jeweiligen Wicklung daran ansteht.

In horizontaler Richtung an die Anlageflächen anschliessend ist die Vorrichtung frei von der Basisplatte nach oben abstehenden Elementen, so dass die Anlageflächen eine einseitige Anlage bilden. Die Vorrichtung so ausgebildet, dass nichts einem Wegbewegen des Führungsdrahtes bzw. Katheters in horizontaler Richtung von der Anlagefläche weg ist entgegensteht.

Der Umstand, dass die Vorrichtung bei jeder Anlagefläche (in horizontaler Richtung senkrecht von der Anlagefläche weg) frei von der Basisplatte nach oben vorstehenden Elementen ist, ist in Fig. 1 beispielhaft an der ersten Anlagefläche durch einen Blockpfeil 66 dargestellt. Der Freiraum (Abstand eventueller Hindernisse) in horizontaler Richtung weg von der Anlagefläche (wie bspw. gemäss Blockpfeil 66) sollte dabei im Verhältnis zu einer Dicke der Wicklung gross sein, d.h. ein Mehrfaches einer erwarteten Wicklungsdicke betragen. Er sollte bspw. mindestens 1 cm, mindestens 2 cm oder mindestens 3 cm betragen; im dargestellten Ausführungsbeispiel ist er unbeschränkt, da in senkrechter Richtung von den Anlageflächen weg gar kein Hindernis angeordnet ist.

Diese Konfiguration hat folgende Vorteile: Erstens können wie erwähnt unterschiedlich grosse Wicklungen stabil abgelegt werden, ohne dass sie aneinander oder an etwas anderes anstossen. Zweitens ist das Entfernen eines medizinischen Führungsdrahtes besonders einfach. Es muss lediglich eine Schlaufe gegriffen und leicht entlang ihrer kleineren Achse (Bezugszeichen 47 in Fig. 2) entgegen ihrer Eigensteifigkeit zusammengedrückt und dann angehoben werden. Das Zusammendrücken entlang der kleineren Achse von beiden Seiten her löst die Schlaufe von allen drei Anlageflächen, wofür der Umstand, dass die Anlageflächen einseitige Anlageflächen im beschriebenen Sinne sind und nicht bspw. eine klemmende Halterung vorhanden ist, von Bedeutung ist. Auch durch Zusammendrücken nur von einer Seite her und anschliessendes Anheben gelingt das Entfernen des Führungsdrahtes auf einfachste Weise.

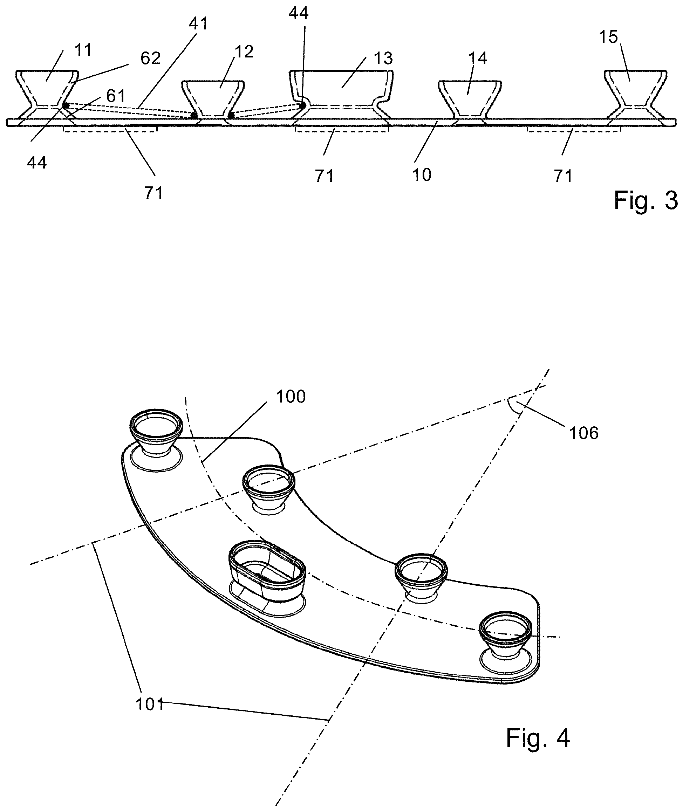

**Figur 3** zeigt eine Seitenansicht der Ablagevorrichtung von Figuren 1 und 2. Das Greifen des Führungsdrahtes kann dadurch erleichtert werden, dass die vorderen Führungselemente, also das erste 11 und das dritte 13 sowie gegebenenfalls das fünfte 15 Führungselement unterseitig, d.h. zur Basisplatte 10 hin, eine auslaufende Partie 61 aufweisen, so dass zusammen mit der überhängenden Partie 62 eine als Hohlkehle ausgebildete Vertiefung für die Führungsdraht-Wicklung 44 gebildet wird, welche Hohlkehle sich in einem vertikalen Abstand zur Basisplatte 10 befindet. Daher kann der Führungsdraht vorderseitig relativ gut untergriffen werden, was die Bedienbarkeit stark erleichtert.

In Fig. 3 sind ausserdem schematisch Klebepartien 71 auf der Unterseite der Basisplatte eingezeichnet. Diese ermöglichen die sehr einfache Befestigung der Ablagevorrichtung auf einem Arbeitstisch. Die Ablagevorrichtung wird mit Vorteil so positioniert werden, dass an ihrer Vorderseite noch genügend Ablagefläche für die Führungsdrahtwicklung vorhanden ist. Die Ablagevorrichtung kann auch in einem flachen Behälter positioniert werden, welches eine entsprechende Flüssigkeit enthält.

Ein weiteres optionales Merkmal ist, dass die Basisplatte 10 als Ganze in der von ihr definierten Ebene gekrümmt ist, was in **Figur 4** illustriert ist. Fig. 4 zeigt die Basisplatte mit gekrümmter Achse 100. Die Krümmung ist so, dass die Hauptachsen 101 der Bahnen in einem von 0° verschiedenen Winkel 106 zueinander stehen, und zwar so, dass die Achsen zum endseitigen Führungselement 12; 14 (entsprechend dem Führungselement, welches in Bezug auf die jeweilige Bahn innen liegt) hin aufeinander zulaufen. Die Richtung der Hauptachsen ist im Wesentlichen unabhängig von der - nicht durch die Vorrichtung definierten - Grösse der Wicklungen und wird definiert durch die Mittelsenkrechte zwischen den Anschlagflächen 21, 23; 24, 26 der vorderseitigen Führungselemente 11, 13, 15, welche Mittelsenkrechte gleichzeitig durch die Anschlagfläche 22, 25 des jeweiligen endseitigen Führungselements führt. Der Winkel 106 zwischen Achsen benachbarter Bahnen beträgt beispielsweise zwischen 10° und 120° oder zwischen 20° und 120°; er kann auch bis zu 180° betragen (d.h. die Ablagevorrichtung definiert dann genau zwei Ablageorte, einander gegenüberliegend).

Dieses Konzept der Anordnung der Führungselemente so, dass die Achsen der Bahnen einen Winkel zueinander bilden, kann auch verwendet werden wenn mehr als die gezeichneten zwei Ablageorte (zwei Bahnen) definiert sind, bis hin zu einer Vorrichtung die einen Kreis oder Kreisring bildend eine Mehrzahl von um einen Mittelpunkt herum angeordneten Ablageorten aufweist.

Das Konzept der Anordnungen so, dass die Achsen der Bahnen einen Winkel bilden, trägt dazu bei, dass die benachbarten Führungsdraht-Wicklungen trotz kompakter Vorrichtung und kompakter Anordnung sauber und in genügendem Abstand voneinander getrennt sind.

Weitere optionale Merkmale der Ablagevorrichtung, welche in Kombination mit den vorstehend beschriebenen Merkmalen oder auch unabhängig davon, und in Kombination miteinander oder unabhängig voneinander, realisierbar sind, umfassen:
- Die Führungselemente sind becherartig ausgestaltet, d.h. sie sind innen hohl (Hohlraum 31, siehe Fig.1). Dieses Merkmal trägt insbesondere zu einer material- und gewichtssparenden Ausgestaltung bei.
- Die Führungselemente 11, 12, 14, 15, die nur einem einzigen Ablageort zugeordnet sind, sind um eine Achse senkrecht zur Ablageebene ungefähr rotationssymmetrisch, d.h. sie haben einen runden Grundriss. Auch das trägt zu einer kompakten Bauweise bei.
- Ein mittleres Führungselement 13 bildet Anschlagflächen 23, 24 von zwei benachbarten Ablageorten, dient also als gemeinsames Führungselement für die zwei benachbarten Ablageorte. Dieses optionale Merkmal (es könnte auch pro Ablageort je ein Führungselement vorhanden sein) trägt zu einer besonders einfachen und kompakten Konstruktion bei. Es kann wie in den Figuren dargestellt optional vorgesehen sein, dass ein solches gemeinsames Führungselement breiter ist, d.h. senkrecht zur Richtung der Bahnen eine grössere Ausdehnung hat als diejenigen Führungselemente, die nur einem Ablageort zugeordnet sind.
- Die Basisplatte 10 und beispielsweise die Vorrichtung als Ganzes können transparent sein. Das ermöglicht beispielsweise das Anbringen von Beschriftungen oder ähnlich an der Unterseite der Basisplatte, ohne dass die Oberseite beeinträchtigt würde.

## Patentansprüche

1. Ablagevorrichtung (1) für mindestens einen aufgewickelten medizinischen Führungsdraht oder Katheter, aufweisend eine Basisplatte (10), welche an einer Plattenoberseite eine Ablageebene definiert sowie von der Basisplatte zur Plattenoberseite abstehend eine Mehrzahl von Führungselementen (11, 12,13), die eine erste, zweite und dritte Anlagefläche (21, 22, 23) für den Führungsdraht bzw. Katheter ausbilden, wobei die erste, zweite und dritte Anlagefläche in einem Schnitt senkrecht zur Ablageebene einen Überhang ausbilden, wobei die erste, zweite und dritte Anlagefläche so angeordnet und orientiert sind, dass sich eine gekrümmte Bahn (41) für den aufgewickelten Führungsdraht bzw. Katheter ergibt, **dadurch gekennzeichnet, dass** eine Wicklung des Führungsdrahts bzw. Katheters, wenn sie entlang der Bahn (41) geführt ist, aufgrund ihrer Eigensteifigkeit an der ersten, zweiten und dritten Anlagefläche ansteht und die erste und die dritte Anlagefläche (21, 23) aussenseitig und die zweite Anlagefläche (22) innenseitig der Bahn angeordnet sind, und dass die Wicklung aufgrund ihrer Eigensteifigkeit nach aussen gegen die erste und die dritte Anlagefläche (21, 23) und nach innen gegen die zweite Anlagefläche (22) gedrückt wird.

2. Ablagevorrichtung nach Anspruch 1, aufweisend mindestens drei von der Plattenoberseite abstehende Führungselemente, wobei die erste Anlagefläche (21) durch ein erstes Führungselement (11), die zweite Anlagefläche (22) durch ein zweites Führungselement (12) und die dritte Anlagefläche (23) durch ein drittes Führungselement (13) gebildet sind, wobei die Bahn (41) innenseitig am ersten Führungselement (11), aussenseitig am zweiten Führungselement (12) und innenseitig am dritten Führungselement entlang führt.

3. Ablagevorrichtung nach Anspruch 1 oder 2, wobei der entlang der Bahn geführte aufgewickelte Führungsdraht bzw. Katheter punktkontaktartig an der ersten, zweiten und dritten Anlagefläche anliegt.

4. Ablagevorrichtung nach einem der Ansprüche 1-3, wobei die Anlageflächen im Schnitt parallel zur Ablagefläche konvex gekrümmt sind.

5. Ablagevorrichtung nach einem der vorangehenden Ansprüche, wobei mindestens im Bereich der ersten und dritten Anlagefläche eine auslaufende Partie (61) vorhanden ist, so dass zusammen mit dem Überhang eine Vertiefung gebildet wird, in welche der aufgewickelte Führungsdraht bzw. Katheter aufgrund seiner Eigensteifigkeit gedrückt wird, welche Vertiefung sich aufgrund der auslaufenden Partie (61) in einem vertikalen Abstand zur Plattenoberseite befindet.

6. Ablagevorrichtung nach einem der vorangehenden Ansprüche, aufweisend eine vierte, fünfte und sechste Anlagefläche (24, 25, 26) für einen zweiten aufgewickelten Führungsdraht oder Katheter, der entlang einer zweiten gekrümmten Bahn (42) geführt ist.

7. Ablagevorrichtung nach Anspruch 6, wobei die dritte und die vierte Anlagefläche (23, 24) durch ein gemeinsames Führungselement (13) gebildet sind.

8. Ablagevorrichtung nach Anspruch 7, wobei das gemeinsame Führungselement (13) senkrecht zu einer Bahnrichtung eine grössere Ausdehnung hat als Führungselemente (11, 12, 14, 15) mit nur einer Anlagefläche.

9. Ablagevorrichtung nach einem der Ansprüche 6-8, wobei die Anlagefläche so angeordnet sind, dass Hauptachsen (101) der Bahnen aufgrund einer Führungsdraht- oder Katheter-Eigensteifigkeit ungefähr ersten und zweiten Bahn in einem von 0° verschiedenen Winkel zueinander stehen.

10. Ablagevorrichtung nach einem der vorangehenden Ansprüche, aufweisend einen Kleber (71) auf einer der Plattenoberseite gegenüberliegenden Plattenunterseite, zum Befestigen der Basisplatte an einer Arbeitsfläche eines Arbeitstischs.

11. Ablagevorrichtung nach einem der vorangehenden Ansprüche, die frei ist von einer seitlichen Wand, welche eine Länge einer Wicklung des medizinischen Führungsdrahts bzw. Katheters begrenzen würde.

12. Ablagevorrichtung nach einem der vorangehenden Ansprüche, wobei mindestens die Basisplatte transparent ist.

13. Verwendung einer Ablagevorrichtung nach einem der vorangehenden Ansprüche für die Ablage eines entgegen einer Eigensteifigkeit-bedingten elastischen Gegenkraft biegbaren medizinischen Führungsdrahtes oder Katheters, wobei der Führungsdraht bzw. Katheter aufgewickelt und anschliessend so abgelegt wird, dass er aufgrund der Eigensteifigkeit an der ersten, zweiten und dritten Anlagefläche ansteht und nach aussen gegen die erste und die dritte Anlagefläche (21, 23) und nach innen gegen die zweite Anlagefläche (22) gedrückt wird.

## Claims

1. Deposition device (1) for at least one wound medical guide wire or catheter, comprising a base plate (10) which defines a deposition plane on a plate upper side and a plurality of guide elements (11, 12, 13) projecting from the base plate towards the plate upper side, which guide elements form a first, second and third contact surface (21, 22, 23) for the guide wire or catheter, wherein the first, second and third contact surfaces form an overhang in a section perpendicular to the storage plane, wherein the first, second and third contact surfaces are arranged and oriented such that a curved path (41) is formed for the wound guide wire or catheter, **characterized in that** when the guide wire or catheter is guided along the path (41), it abuts against the first, second and third contact surfaces due to its inherent rigidity, and the first and third contact surfaces (21, 23) are arranged on the outside and the second contact surface (22) on the inside of the path, and that the winding is pressed outwardly against the first and third contact surfaces (21, 23) and inwardly against the second contact surface (22) due to its inherent rigidity.

2. Deposition device according to claim 1, comprising at least three guide elements projecting from the top of the plate, wherein the first contact surface (21) is formed by a first guide element (11), the second contact surface (22) by a second guide element (12) and the third contact surface (23) by a third guide element (13), wherein the web (41) runs along the inside of the first guide element (11), along the outside of the second guide element (12) and along the inside of the third guide element.

3. Deposition device according to claim 1 or 2, wherein the wound guide wire or catheter guided along the track rests in point contact on the first, second, and third contact surfaces.

4. Deposition device according to one of claims 1-3, wherein the contact surfaces are convexly curved in section parallel to the deposition plane.

5. Deposition device according to one of the preceding claims, wherein at least in the region of the first and third contact surfaces there is a run-out portion (61) such that, together with the overhang, a recess is formed into which the wound guide wire or catheter is pressed due to its inherent rigidity, which recess is located at a vertical distance from the plate upper side due to the run-out portion (61). catheter is pressed into due to its inherent rigidity, which recess is located at a vertical distance from the top of the plate due to the run-out portion (61).

6. Deposition device according to one of the preceding claims, comprising a fourth, fifth and sixth contact surface (24, 25, 26) for a second wound guide wire or catheter which is guided along a second curved path (42).

7. Deposition device according to claim 6, wherein the third and fourth contact surfaces (23, 24) are formed by a common guide element (13).

8. Deposition device according to claim 7, wherein the common guide element (13) has a greater extension perpendicular to a path direction than guide elements (11, 12, 14, 15) with only one contact surface.

9. Deposition device according to one of claims 6-8, wherein the contact surfaces are arranged such that main axes (101) of the paths are at an angle to each other other than 0° due to the inherent rigidity of the guide wire or catheter, approximately corresponding to the first and second paths.

10. Deposition device according to one of the preceding claims, comprising an adhesive (71) on a plate underside opposite the plate top side for fastening the base plate to a work surface of a work table.

11. Deposition device according to one of the preceding claims, which is free of a side wall that would limit the length of a winding of the medical guide wire or catheter.

12. Deposition device according to one of the preceding claims, wherein at least the base plate is transparent.

13. Use of a deposition device according to one of the preceding claims for depositing a medical guide wire or catheter that can be bent against an elastic counterforce caused by its own rigidity, wherein the guide wire or catheter is wound up and then deposited in such a way that, due to its inherent rigidity, it rests against the first, second, and third contact surfaces and is pressed outward against the first and third contact surfaces (21, 23) and inward against the second contact surface (22).

## Revendications

1. Dispositif de rangement (1) pour au moins un fil guide médical enroulé ou un cathéter, comprenant une plaque de base (10) qui définit un plan de rangement sur une face supérieure de la plaque, ainsi qu'une pluralité d'éléments de guidage (11, 12, 13) qui font saillie de la plaque de base vers la face supérieure de la plaque et qui forment une première, une deuxième et une troisième surface d'appui (21, 22, 23) pour le fil-guide ou le cathéter, les première, deuxième et troisième surfaces d'appui formant, dans une section perpendiculaire au plan de rangement, un surplomb, les première, deuxième et troisième surfaces d'appui étant disposées et orientées de telle sorte qu'il en résulte une trajectoire courbe (41) pour le fil-guide ou le cathéter enroulé, **caractérisé en ce que**, lorsqu'il est guidé le long de la trajectoire (41), le fil-guide ou le cathéter, en raison de sa rigidité propre, s'appuie sur les première, deuxième et troisième surfaces d'appui, et les première et troisième surfaces d'appui (21, 23) sont disposées à l'extérieur et la deuxième surface d'appui (22) à l'intérieur de la trajectoire, et que l'enroulement est pressé vers l'extérieur contre les première et troisième surfaces d'appui (21, 23) et vers l'intérieur contre la deuxième surface d'appui (22) en raison de sa rigidité propre.

2. Dispositif de rangement selon la revendication 1, comportant au moins trois éléments de guidage faisant saillie de la face supérieure de la plaque, la première surface d'appui (21) étant formée par un premier élément de guidage (11), la deuxième surface d'appui (22) par un deuxième élément de guidage (12) et la troisième surface d'appui (23) par un troisième élément de guidage (13), la bande (41) s'étendant à l'intérieur le long du premier élément de guidage (11), à l'extérieur le long du deuxième élément de guidage (12) et à l'intérieur le long du troisième élément de guidage.

3. Dispositif de rangement selon la revendication 1 ou 2, le fil de guidage ou cathéter enroulé guidé le long de la piste reposant par points sur les première, deuxième et troisième surfaces d'appui.

4. Dispositif de rangement selon l'une des revendications 1 à 3, les surfaces d'appui étant courbées de manière convexe dans la section parallèle à la surface de rangement.

5. Dispositif de rangement selon l'une des revendications précédentes, dans lequel au moins dans la zone de la première et de la troisième surface d'appui, il existe une partie en décroissance (61) de telle sorte qu'avec le surplomb, il se forme un renfoncement dans lequel le fil de guidage ou le cathéter enroulé est pressé en raison de sa rigidité propre, lequel renfoncement se trouvent, grâce à la partie en décroissance (61), à une distance verticale de la face supérieure de la plaque.

6. Dispositif de rangement selon l'une des revendications précédentes, comportant une quatrième, une cinquième et une sixième surfaces d'appui (24, 25, 26) pour un deuxième fil-guide ou cathéter enroulé, qui est guidé le long d'une deuxième trajectoire courbe (42).

7. Dispositif de rangement selon la revendication 6, dans lequel les troisième et quatrième surfaces d'appui (23, 24) sont formées par un élément de guidage commun (13).

8. Dispositif de rangement selon la revendication 7, dans lequel l'élément de guidage commun (13) a, perpendiculairement à une direction de trajectoire, une extension plus grande que les éléments de guidage (11, 12, 14, 15) comportant une seule surface d'appui.

9. Dispositif de rangement selon l'une des revendications 6 à 8, dans lequel les surfaces d'appui sont disposées de telle sorte que les axes principaux (101) des bandes forment, en raison de la rigidité propre du fil de guidage ou du cathéter, approximativement un premier et un deuxième angle différents de 0° l'un par rapport à l'autre.

10. Dispositif de rangement selon l'une des revendications précédentes, comportant un adhésif (71) sur une face inférieure de la plaque opposée à la face supérieure de la plaque, pour fixer la plaque de base à une surface de travail d'une table de travail.

11. Dispositif de rangement selon l'une des revendications précédentes, qui est dépourvu d'une paroi latérale qui limiterait la longueur d'un enroulement du fil-guide médical ou du cathéter.

12. Dispositif de rangement selon l'une des revendications précédentes, dans lequel au moins la plaque de base est transparente.

13. Utilisation d'un dispositif de rangement selon l'une des revendications précédentes pour le rangement d'un fil guide médical ou d'un cathéter pouvant être courbé sous l'effet d'une force élastique due à sa rigidité propre, le fil guide ou cathéter est enroulé puis déposé de telle sorte qu'il repose sur les première, deuxième et troisième surfaces d'appui en raison de sa rigidité propre et qu'il soit pressé vers l'extérieur contre les première et troisième surfaces d'appui (21, 23) et vers l'intérieur contre la deuxième surface d'appui (22).
